# EUROPEAN PATENT SPECIFICATION

(11) **EP 3 232 996 B1**
(45) Date of publication and mention of the grant of the patent: **15.07.2020**
(21) Application number: 15816651.2
(22) Date of filing: 17.12.2015
(51) Int. Cl.: A61F 5/442, A61M 3/02

(54) **A SYSTEM AND A METHOD FOR ANAL AND/OR STOMAL IRRIGATION**
SYSTEM UND VERFAHREN ZUR ANAL- UND/ODER STOMA-IRRIGATION
SYSTÈME ET PROCÉDÉ POUR L'IRRIGATION DE L'ANUS ET/OU D'UNE STOMIE

(30) Priority: 19.12.2014 DK 201470806
(43) Date of publication of application: 25.10.2017
(73) Proprietor: Coloplast A/S, 3050 Humblebæk (DK)
(72) Inventor: HVID, Niels, 2950 Vedbaek (DK); HICKMOTT, Richard Morgan, 3000 Helsingoer (DK)
(86) International application number: PCT/DK2015/050402
(87) International publication number: WO 2016/095928

(56) References cited:
- EP-A1- 2 671 602
- EP-A1- 2 679 259
- WO-A1-2011/023196

## Description

The present invention relates to a system and a method for anal and/or stomal irrigation comprising a reservoir for an irrigating liquid, a catheter comprising a catheter tip for insertion into the rectum and/or stoma of a user, and an expandable retention element, such as an expandable balloon, for fixation of the catheter tip within the user's rectum or stoma. The invention provides in particular a valve and tubing system for controlling the supply of the irrigating liquid to the catheter tip as well as the supply and the withdrawal of the irrigating liquid to and from the expandable retention element.

Examples of prior art can be seen in EP 2679259 A1, WO 2011/023196 A1 and EP 2671602 A1.

### Brief Description of the Drawing

The accompanying drawings are included to provide a further understanding of embodiments and are incorporated into and a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.
Fig. 1 shows an embodiment of a system for anal and/or stomal irrigation;
Fig. 2 shows an embodiment of a tubing and valve system of an embodiment of a system for anal and/or stomal irrigation;
Figs. 3-5 show respective embodiments of flow configurations in the tubing and valve system of Fig. 2;
Figs. 5a and 5b show embodiments of flow configurations in an alternative embodiment;
Figs. 6 and 7 illustrate exemplary curves of temperature of an irrigating liquid in a reservoir during filling or refilling of irrigating liquid into the reservoir; and
Fig. 8 illustrates an embodiment of a method for predicting a temperature of an irrigating liquid in a reservoir of a system for anal irrigation; and
Figs. 9A-9E illustrate displays of a user-operable control interface for controlling operation of the system.

### Detailed Description

Control of voluntary bowel functions is frequently limited or absent in patients suffering from certain disabilities, such as spinal injuries, multiple sclerosis or bifid spine. Such lack of control of voluntary bowel functions typically results in faecal incontinence or intractable constipation, as patients have significantly reduced ability to sense the presence of faeces in the colon terminal part and the rectum and to sense the evacuation stimulus. Patients having undergone stomal surgery wherein a catheterizable stoma is constructed may suffer from similar difficulties.

It is known to accomplish bowel emptying by irrigation (i.e. flushing) of the rectum or stoma, by an irrigating fluid, such as tap water or saline, which is provided through an intermittent catheter with a tip which is configured and sized for insertion into the rectum or stoma, where it remains in a fixed position by an expandable inflation element, such as a balloon. The balloon may be inflatable by air or by water. Once the rectum or stoma has been flushed with the irrigating liquid, the expandable retention element is allowed to collapse to its non-deflated state, allowing the catheter to be withdrawn from the rectum or stoma, and allowing the liquid and faeces to evacuate. The catheter is connected to a reservoir of the irrigating liquid through a tube, and a pump may be provided for displacing the irrigating liquid from the reservoir to the catheter.

The development of transanal or transstomal irrigation systems has hitherto focussed on aspects of tubings, catheters and pumps. It is hence an object to improve known systems further, in particular by improving safety and user-convenience in relation to self-irrigation, and more particularly by improving control and operation of the expandable retention element.

Embodiments relate to a system for anal and/or stomal irrigation comprising:
a reservoir for an irrigating liquid;
a catheter comprising a catheter tip for insertion into the rectum and/or stoma of a user and for expelling of the irrigating liquid from the catheter tip, the catheter further comprising an expandable retention element for fixation of the catheter tip within the user's rectum or stoma;
a tubing system providing a first conduit for the irrigating liquid between the reservoir and the catheter and providing a second conduit for the irrigating liquid between the reservoir and the expandable retention element;
a valve system in the second conduit for controlling the flow of the irrigating liquid between the reservoir and the expandable retention element;
a pump operable to pump the irrigating liquid from the reservoir to the catheter tip, the pump comprising a reversible pump, which is operable in one direction to pump the irrigating liquid into the expandable retention element for inflation thereof, and which is operable in a reverse direction to withdraw the irrigating liquid from the expandable retention element for collapsing thereof;
   and
the pump and the valve system being controllable to selectively:
   - pump the irrigating liquid into the expandable retention element for expansion thereof;
   - pump the irrigating liquid through the catheter for expelling of the irrigating fluid from the catheter tip and into the user's rectum or stoma;
   - withdraw the irrigating liquid from the retention element for purging thereof.

Further embodiments relate to a method of operating a system for anal and/or stomal irrigation, said system comprising:
a reservoir for an irrigating liquid;
a catheter comprising a catheter tip for insertion into the rectum and/or stoma of a user and for expelling of the irrigating liquid from the catheter tip, the catheter further comprising an expandable retention element for fixation of the catheter tip within the user's rectum or stoma;
a tubing system providing a first conduit for the irrigating liquid between the reservoir and the catheter and providing a second conduit for the irrigating liquid between the reservoir and the expandable retention element;
a valve system in the second conduit for controlling the flow of the irrigating liquid between the reservoir and the expandable retention element;
a pump operable to pump the irrigating liquid from the reservoir to the catheter tip, the pump comprising a reversible pump, which is operable in one direction to pump the irrigating liquid into the expandable retention element for inflation thereof, and which is operable in a reverse direction to withdraw the irrigating liquid from the expandable retention element for collapsing thereof;
   said method comprising controlling and operating the pump and the valve system to selectively:
   - pump the irrigating liquid into the expandable retention element for expansion thereof;
   - pump the irrigating liquid through the catheter for expelling of the irrigating fluid from the catheter tip and into the user's rectum or stoma;
      withdraw the irrigating liquid from the retention element for purging thereof.

The pump may be manually or electrically driven. The ability of the pump and the valve system to withdraw the irrigating liquid from the retention element for purging thereof allows the retention element to be purged in a controlled manner. Expansion of the retention element as well as collapsing thereof may hence be accurately controlled by adequate control of the pump and the valve system. Collapsing of the retention element by a controlled action, notably by forced purging caused by a pumping action of the pump, enables purging of the retention element even under circumstances, at which the retention element is expanded by a relatively low pressure, which is too low for the irrigating liquid to escape from the retention element merely be opening a valve of the valve system.

The pump is preferably an electrically driven pump, and the pump and the valve system are preferably controllable by an electronic control system.

The valve system and the tubing system may be configurable to withdraw the irrigating liquid from the retention element during purging thereof by conveying the irrigating liquid from the retention element directly into user's rectum or stoma without the irrigating liquid passing into or through the reservoir. This incurs several benefits. Firstly, the user is released from encountering the possibly disturbing or uncomfortable experience of noting that irrigating liquid passes from the retention element, which is fixed in the user's rectum or stoma, back to the reservoir. Accordingly, user comfort and trust in the system is improved. Secondly, as a temperature equilibrium will be achieved between the irrigating liquid within the expanded retention element and the user's body while the retention element is fixed within the bowel, the irrigating liquid used for expansion may conveniently be used for irrigation (i.e. flushing of the bowel) without the need for any further temperature management for that part of the irrigating liquid. Thirdly, the distance to be travelled by the irrigating liquid and hence power consumption of the pump may be minimized when the irrigating liquid is allowed to pass directly from the retention element to the catheter tip for irrigation of the bowel.

Alternatively, however, the pump, the valve system and the tubing system may be configurable to withdraw the irrigating liquid from the retention element during purging thereof by conveying the irrigating liquid from the retention element into the reservoir.

In one embodiment, the pump comprises an electrical pump, which is reversible and thereby operable in one direction to pump the irrigating liquid into the expandable retention element for inflation thereof, and which is operable in a reverse direction to withdraw the irrigating liquid from the expandable retention element for collapsing thereof. The ability of the pump to pump irrigating liquid into the retention element and to withdraw the irrigating liquid therefrom may thus be accomplished in an inexpensive manner by a pump, which is easy to operate and control.

The valve system within the tubing system is preferably configured to selectively cause one flow configuration selected from a first, second and third flow configuration at a time, wherein:
- the first flow configuration is arranged to cause a transfer of the irrigating liquid, by means of said pump, from the reservoir into the expandable retention element;
- the second flow configuration is arranged to transfer the irrigating liquid, by means of said pump, from the reservoir to the catheter;
- the third flow configuration is arranged to transfer the irrigating liquid, by means of said pump, away from the expandable retention element.

Thus, in the first flow configuration the irrigating liquid is transferred from the reservoir to the expandable retention element for expansion thereof. In the second flow configuration, the irrigating liquid is transferred from the reservoir to the catheter, i.e. to the catheter tip for insertion into the user's rectum or stoma. In the third flow configuration, the irrigating liquid is transferred away from the expandable retention element, either directly to the catheter tip for flushing of the user's bowel without the irrigating liquid passing into or through the reservoir, or back to the reservoir.

As a safety measure, to prevent rupture of the expandable retention element due to overpressure and/or to prevent the exertion of overpressure to the user's bowel, a first relief valve may be provided, the valve being configured to open if pressure in a bowel of the user exceeds a first threshold limit in the first flow configuration, i.e. during expansion of the expandable retention element. Preferably, when the first relief valve opens, an amount of the irrigating liquid is transferred to the reservoir or expelled into a toilet facility if the valve, for instance, is placed in a connect portion of the catheter.

A second relief valve may be provided as a further or alternative safety measure, the second relief valve being configured to open if pressure in a bowel of the user exceeds a second threshold limit in the second flow configuration, i.e. during flushing of the user's bowel, so as to transfer an amount of the irrigating liquid to the reservoir or expel it into a toilet rather than keeping pumping irrigating liquid into the user's rectum or stoma.

In general, it may be desirable to transfer liquid to be expelled due to overpressure out of the system, i.e. into a toilet facility, rather than into the system itself, such as into the reservoir.

Generally, the valve and tubing system may be operable to redirect the irrigating liquid to the reservoir if the pressure within the expandable retention element exceeds a predetermined threshold level.

In order to further control the supply of the irrigating liquid to the expandable retention element and/or to the catheter, the system may comprise:
a first actively controllable valve arranged in the tubing and/or the catheter at a position between the pump and the expandable retention element; and/or
a second actively controllable valve arranged in the tubing at a position between the pump and the catheter.

The actively controllable valves are operable in order to achieve the desired one of the first, second and third flow configuration. More specifically, when the first actively controllable valve is open and the second one is closed, irrigating liquid may pass to the expandable retention from the reservoir, or away from the expandable retention element. In the state wherein the first valve is open and the second one is closed, the direction of flow through the pump may be controllable by the direction of rotation of the pump motor. The destination of irrigating liquid forced away from the expandable retention element may be selected by one or more passively controllable valves, such as check valves, or by one or more actively controllable valves. When the first actively controllable valve is closed and the second one is open, irrigating liquid may pass from the reservoir to the catheter without entering the expandable retention element.

At least one check valve is preferably provided for preventing a backflow of the irrigating liquid from the pump in a direction towards the reservoir, so as to force liquid withdrawn from the expandable retention element towards the catheter and into the user's rectum or stoma.

A user-operable control interface may be provided for controlling operation of the valve system and/or the pump. The user may for example select valve settings to select a flow configuration among the above-mentioned first, second and third flow configurations, and the user may further set operating parameters of the system, such as expansion pressure of the retention element, or an operating speed of the pump, i.e. flow rate of irrigating liquid for irrigation, or an irrigation duration.

In one embodiment, the pump is operable to repeatedly expand and collapse so as to stimulate the peristaltic of the user's bowel. Such action of the pump may be activatable by the user through the control interface. Its settings, such as duration or frequency of repeated expansion and collapsing may be defined through the interface. The expandable retention element may be incrementally expandable or collapsible, allowing the user to control expansion or collapsing of the retention element in response to the user's sensation of the state of expansion.

The control system may be configured to control a flow condition of the irrigating liquid at the catheter tip during anal or stoma irrigation. The control system may hence comprise a controller for controlling operation of the pump, at least one sensor for determining a measure of pressure at at least one first predetermined position in the tubing system and/or the catheter during operation of the pump, and a processor for determining or estimating said flow condition at the catheter tip on the basis of said measure of pressure. Further, the control system may be configured to control the pumping operation of the pump in response to said measure of pressure.

The provision of the at least one sensor for determining a measure of pressure at at least one first predetermined position in the tubing system and/or the catheter during operation of the pump allows the processor to determine or estimate a flow condition at the catheter tip on the basis of such measure. For example, the rise of the pressure at a particular flow restrictor within the tubing system to a predetermined level may indicate the presence of irrigating liquid at the tip of the catheter. Similarly, the rise of pressure at the catheter tip itself may indicate the presence of irrigating liquid at the tip.

In one embodiment, the control system may comprise a memory for storing at least one pressure threshold value indicative of the presence of the irrigating liquid at at least the first predetermined position in the tubing system and/or the catheter and/or at at least one second predetermined position in the tubing system and/or the catheter. In such an embodiment, the control system may be configured to continue the pumping operation of the pump for a limited period of time after determination, by the at least one sensor, of a pressure value at the at least one first predetermined position which is at least equal to the pressure threshold value or a value derived therefrom. For example, one of the first and second predetermined positions may be a position at the catheter tip or in the vicinity thereof, in which case the control system may be configured to continue said pumping operation for a certain duration after the determination of said pressure threshold value. Accordingly, the amount of irrigating liquid expelled from the catheter tip may be accurately controlled by control of said duration.

A thermo sensor may further be provided, which is connected to the reservoir for obtaining a measure of a temperature within the reservoir, the tubing system and/or the catheter. The control system may be operatively connected with the thermo sensor, and the control system may be configured to determine a temperature within the reservoir before the irrigating liquid is filled or re-filled into the reservoir, determine an initial change of the temperature within the reservoir upon commencement of filling or refilling of the irrigating liquid into the reservoir, and predict a future asymptotic value of the temperature within the reservoir on the basis of at least the initial change. The control system may further be configured to continuously determine a current temperature or a current rate of change of the temperature within the reservoir while the irrigating liquid is filled or refilled into the reservoir, and to continuously update the prediction of the future asymptotic value of the temperature within the reservoir on the basis of at least said current temperature and/or rate of change of the temperature.

Thanks to the thermo sensor and the control system, a prediction of the future asymptotic value of the temperature within the reservoir once filled, notably of the irrigating liquid, may be made. As the prediction of the future asymptotic temperature value is continuously updated on the basis of the current temperature and/or the rate of change of temperature, a change of temperature of the liquid supplied to the reservoir, such as for example a change of the ratio between hot and cold tap water, is adequately reflected in the temperature prediction. The temperature prediction may be communicated to the user, e.g. via a display of the system, thus allowing the user to ascertain if the temperature of the supplied liquid, typically tap water, is to be increased or decreased.

### Detailed Description of the Drawing

Embodiments, and features of the various exemplary embodiments described in this application, may be combined with each other ("mixed and matched"), unless specifically noted otherwise.

The invention, however, is only defined by the scope of the appended claims.

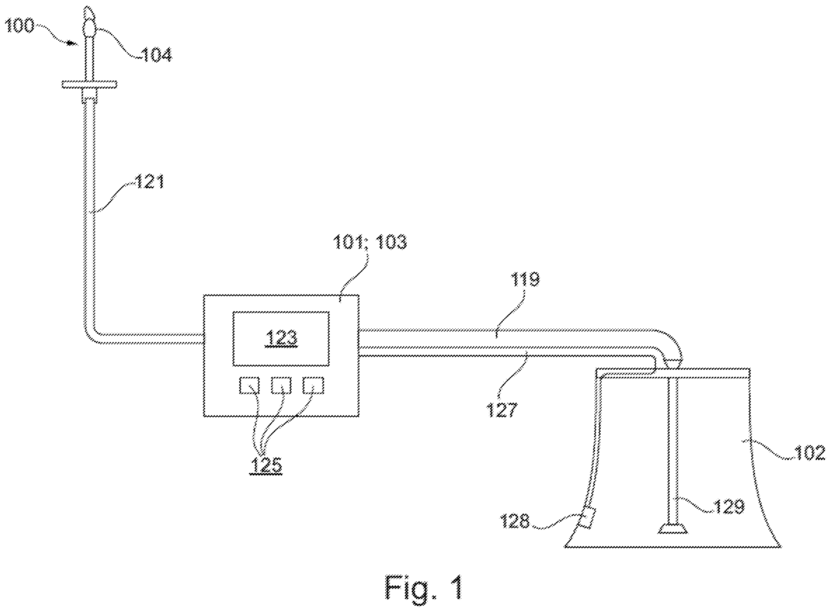

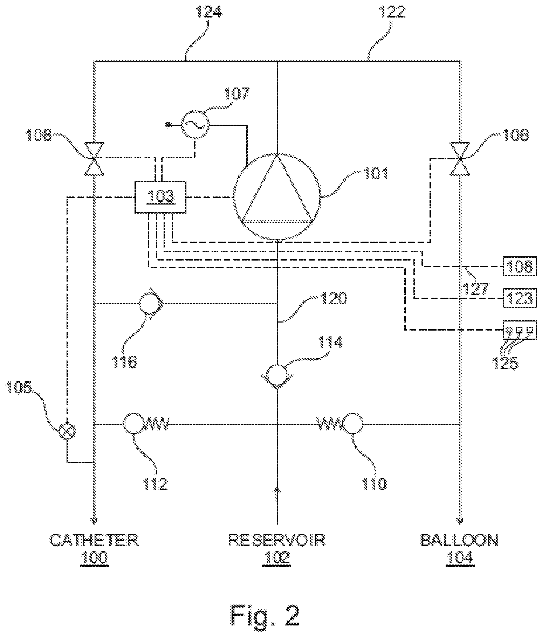

Fig. 1 shows an embodiment of a system for anal and/or stomal irrigation. The system comprises a catheter 100 sized and configured for insertion into the rectum or stoma of a user. A housing for a pump 101 is provided for transferring an irrigating liquid contained within a reservoir 102 to the catheter 100 and to an expandable retention element 104 in the form of a balloon configured to fixate the catheter within the user's rectum or stoma. A control system 103 for the pump and a valve system (not visible in Fig. 1) is further housed within the housing of the pump 101. Tube portion 119 connects the reservoir 102 to the pump 101, and tube portion 121 connects the pump within the housing of the pump 101 to the catheter 100 and expandable retention element 104. As discussed in further detail in relation to Figs. 2-5 below, tube portion 121 includes separate conduits for connecting the pump to the catheter for expelling of irrigating liquid from the catheter tip and for expansion of the balloon 104, respectively. Tube portion 119 attaches to dip tube 129 for sucking irrigating liquid from the reservoir 102. The housing of the pump 101 is provided with a display 123 for communicating an operating state of the system and/or an asymptotic temperature value to the user, and user-operable control buttons 125 are provided as part of a user operable control interface for controlling operation of the valve system (not visible in Fig. 1) and/or pump 101. A thermo sensor 128 attaches to a wall of the reservoir 102, a wired connection 127 being provided for communicating a signal from the thermo sensor 128 to the control system 103 within housing of the pump 101.

Fig. 2 illustrates an embodiment of a tubing and valve system of the system of Fig. 1. As shown, pump 101 is connected to reservoir 102 via conduit 120 comprising a first check valve. The conduit 120 is provided within tube portion 119 (see Fig. 1). The first check valve 114 may be provided within tube portion 119, or within the housing of the pump 101, or within the dip tube 129. Downstream of the pump (when seen in flow direction from the reservoir towards the catheter 100 and the balloon 104), the tubing system has two branches, one of which includes conduit 122 connecting to the balloon 104 via a first actively controllable valve 106. The conduit 122 is provided within tube portion 121. The first actively operable valve 106 may be provided within the tube portion 121, or within the catheter 100, or within the housing of the pump 101. The other branch of the tubing system downstream of the pump includes conduit 124 connecting to the catheter 100 via a second actively controllable valve 108. The conduit 124 is provided within tube portion 121. The second actively operable valve 108 may be provided within the tube portion 121, or within the catheter 100, or within the housing of the pump 101. As shown by dashed lines in Figs. 2-5, the actively controllable valves 106 and 108 are controllable by the control system 103.

A pressure sensor 105 is provided for measuring pressure at least one first predetermined position in the tubing system 119, 120, 121, 122, 124 and/or the catheter 100 during operation of the pump 101. The pressure sensor 105 outputs a signal to the control system 103, which operates the pump and/or the actively controllable valves 106, 108 on the basis of said signal and other signals as described herein. The control system 103 includes a processor for determining or estimating a flow condition at the catheter tip on the basis of the measure of pressure provided by pressure sensor 105, and the control system is configured to control the pumping operation of the pump in response to said measure of pressure. More specifically, the control system continues pumping operation of the pump 101 for a limited period of time after determination, by the pressure sensor 105, of a pressure value which is at least equal to a pressure threshold value or a value derived therefrom. Thus, the amount of irrigating liquid expelled from the catheter tip may be accurately controlled. In the embodiment shown, the pressure sensor 105 is arranged in the tubing system 121, 124 in the vicinity of the catheter 100 or within the catheter 100 itself.

Control system 103 further receives input from user-operable control buttons 125, and thermo sensor 128, and control system 103 communicates data to display 123. The data communicated to display 123 may include a predicted future asymptotic value of the temperature of the irrigating liquid within the reservoir 102 as determined by thermo sensor 128. The data may be continuously updated as the control system 103 continuously updates the temperature prediction while irrigating liquid is being filled or re-filled into the reservoir.

First and second relief valves 110 and 112 are provided for allowing irrigating liquid to escape from the balloon 104 or from the catheter 100 in case the pressure therein exceeds the threshold pressure defined by the relief valves. The first relief valve 110 drains liquid from the balloon 104 to the reservoir 102 in the case of excessive pressure within the balloon 104, and the second relief valve drains liquid from the catheter 100 to the reservoir 102 in the case of excessive pressure within the user's rectum or stoma.

Further, first and second check valves 114 and 116 are provided for preventing undesired backflow of liquid in the tubing system. The first check valve 114 is provided within conduit 120 between the pump 101 and the reservoir 102 in order to prevent backflow of irrigating liquid from the pump 101, or any position downstream of the pump, to the reservoir 102. The second check valve 116 is provided in a side branch in the tubing system connecting conduit 124 to conduit 120. The first and second check valves 114 and 116 may be provided within tube portions 119 and 121 (see Fig. 1) or within the housing of the pump 101, or alternatively the first check valve 114 may be provided in dip tube 129. Second check valve 116 may be provided within the catheter 100.

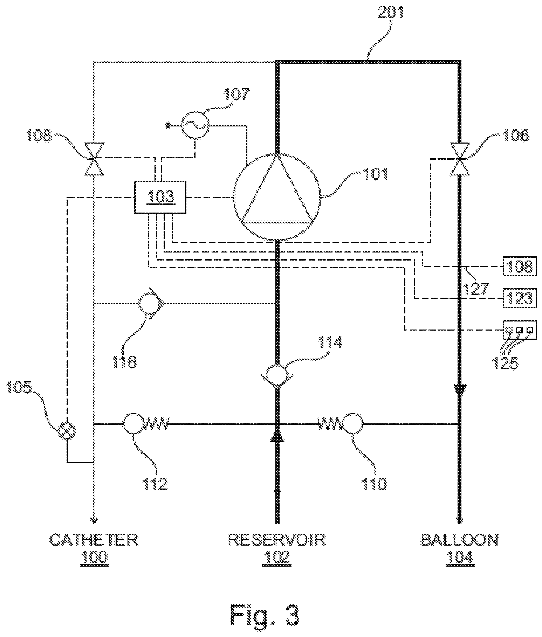

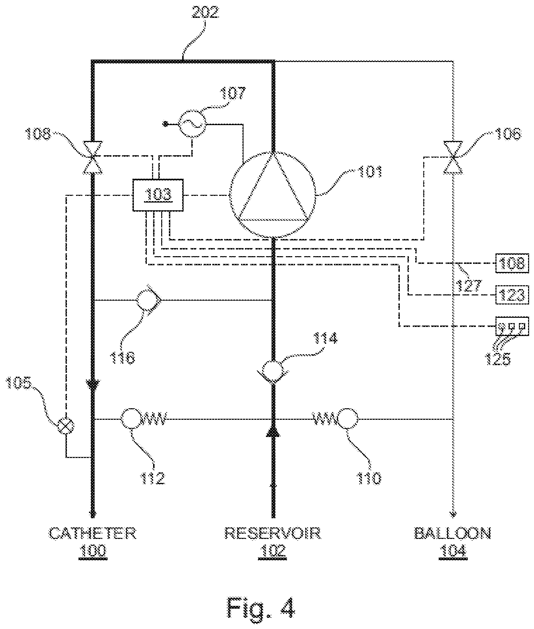

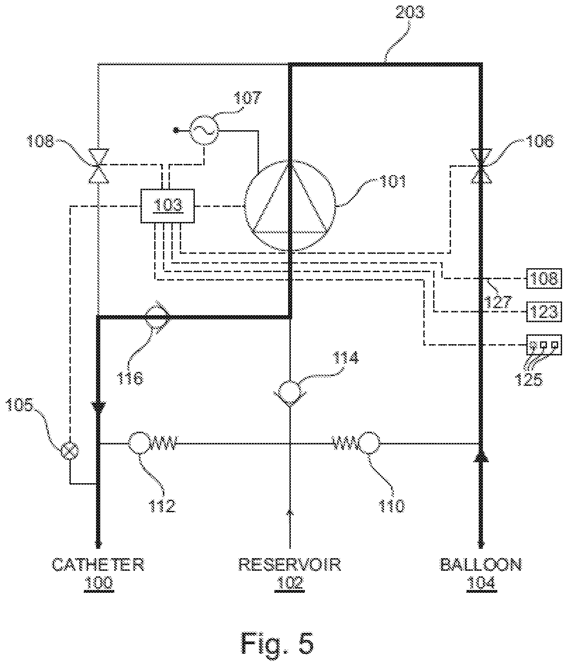

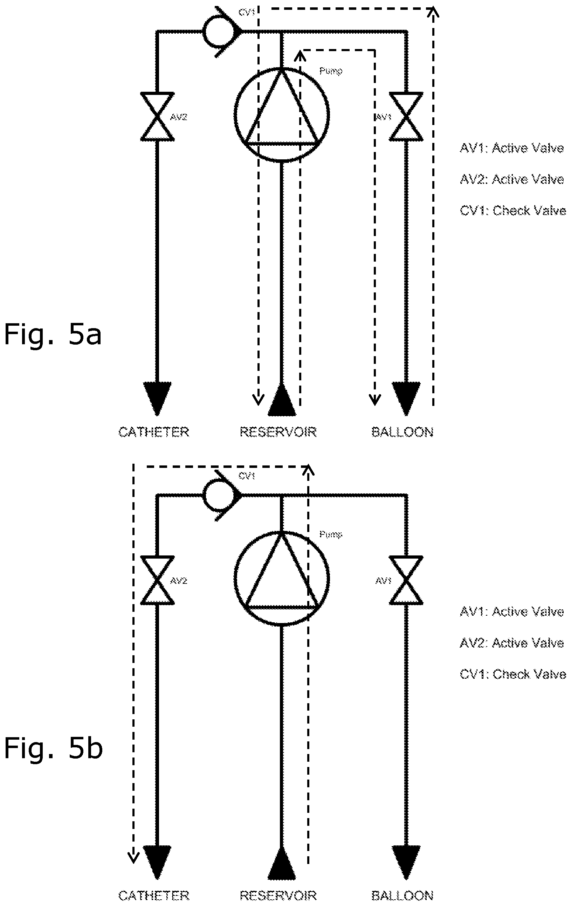

Figs. 3-5 show respective embodiments of flow configurations in the tubing and valve system of Fig. 2. In the first flow configuration 201 shown in Fig. 3, the first actively controllable valve 106 is open, and the second actively controllable valve 108 is closed while the pump 101 operates. Accordingly, irrigating liquid is transferred from the reservoir 102 to the balloon 104 for expansion thereof. In the second flow configuration 202 shown in Fig. 4, the second actively controllable valve 108 is open, and the first actively controllable valve 106 is closed while the pump 101 operates. Irrigating liquid is thus transferred from the reservoir 102 to the catheter 100, at the tip of which the liquid is expelled into the user's rectum or stoma so as to irrigate the user's bowel. In the third flow configuration 203 shown in Fig. 5, operation of the pump 101 is reversed, and the first actively controllable valve 106 is open, while the second actively controllable valve 108 is closed. The balloon 104 is hence purged, and the irrigating liquid withdrawn therefrom flows from the balloon 104 to the catheter 100, at the tip of which it is expelled.

In the alternative embodiment of Figs. 5a and 5b, in which the balloon 104 may be emptied into the reservoir 102 by forced action of the pump 101. The dashed lines in Figs. 5a and 5b indicate respective flow configurations for expanding the balloon and purging thereof into the reservoir. Fig. 5b indicates a flow configuration for expelling of the irrigating liquid through the catheter. In the flow configurations of Fig. 5a, a first actively controlled valve AV1 is open, and a second actively controlled valve AV2 is closed. For expansion of the balloon, the pump 101 operates in a first operating direction, whereas for purging, i.e. collapsing of the balloon, the pump 101 operates in a second operating direction opposite to the first operating direction. In the flow configuration of Fig. 5b, the first actively controlled valve AV1 is closed, and the second actively controlled valve AV2 is open. Check-valve CV1 prevents backflow of the irrigating liquid from the catheter tubing conduit towards the reservoir.

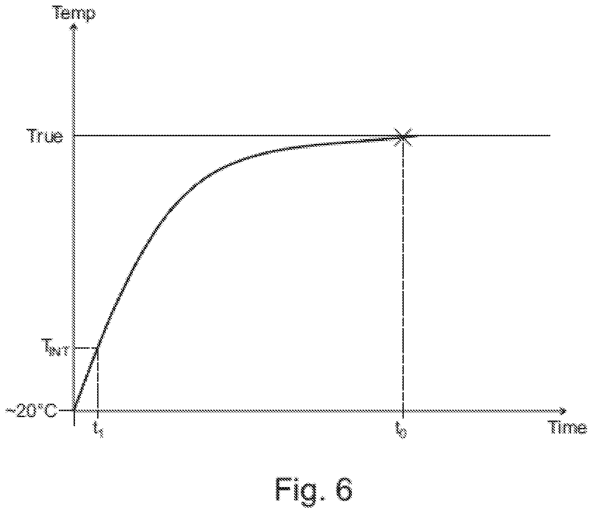

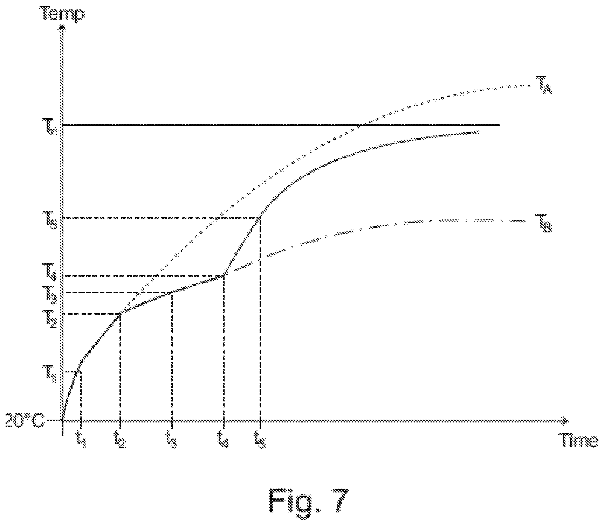

Figs. 6 and 7 illustrates exemplary curves of temperature of the irrigating liquid in the reservoir 102 during filling or refilling of irrigating liquid into the reservoir. In the chart of Fig. 6, the initial temperature of the irrigating liquid within the reservoir 102 as determined by thermo sensor 128 is approximately 20°C. As the user's bowel should be irrigated with liquid at a temperature not exceeding approximately 40°C, preferably at a temperature of 20-40°C, most preferably at a temperature of 36-38°C, the user starts pouring liquid, such as tap water, at an elevated temperature into the reservoir.

Next, an initial change of the temperature within the reservoir is determined by the thermo sensor 128 upon commencement of filling or refilling of the irrigating liquid into the reservoir 120. In Fig. 6, the initial temperature change is represented by elevated temperature TINT at time t1. On the basis of the initial temperature change, a future asymptotic value of the temperature, denoted "True" in Fig. 6, within the reservoir is predicted on the basis of at least the initial change.

As shown in Fig. 7, a current temperature or a current rate of change of the temperature within the reservoir is continuously determined by means of thermo sensor 128 and control system 103, while the irrigating liquid is filled or refilled into the reservoir, and the prediction of the future asymptotic value of the temperature within the reservoir is continuously updated on the basis of at least said current temperature and/or rate of change of the temperature. More specifically, at the start of the filling or refiling procedure, an initial temperature change T1 is determined at a first point in time, t1. The first initial temperature change as represented by T1 is used for a first prediction, TA, of a future asymptotic temperature value of the irrigating liquid within the reservoir 102 once filled. At a second point in time, t2, when the temperature as determined by the thermo sensor 128 has reached level T2, the temperature of the liquid supplied to the reservoir changes, for example as the user changes the ratio of hot to cold water in the tap. At a third point in time t3, a third temperature value T3 is obtained, and second prediction TB is made. Subsequently, at a fourth point in time, t4, a fourth temperature level T4 is reached, and the temperature of the liquid filled into the reservoir 102 changes abruptly for a second time. The change of the supplied liquid is reflected by temperature T5 at time t5, on the basis of which a third asymptotic temperature prediction T∞ is made.

During the above procedure, the predicted temperature values TA, TB and T∞ are shown to the user via display 123 (see Figs. 1-5) as they are determined by the control system 103.

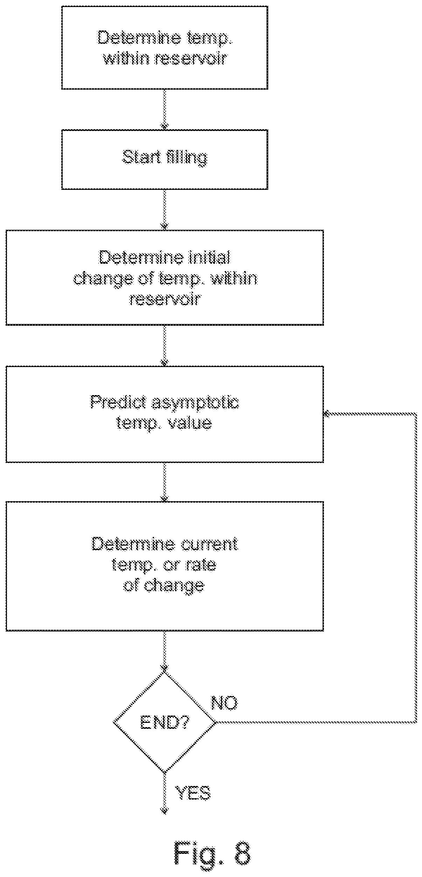

The above procedure of continuously determining and updating the asymptotic temperature prediction is generally depicted in Fig. 8.

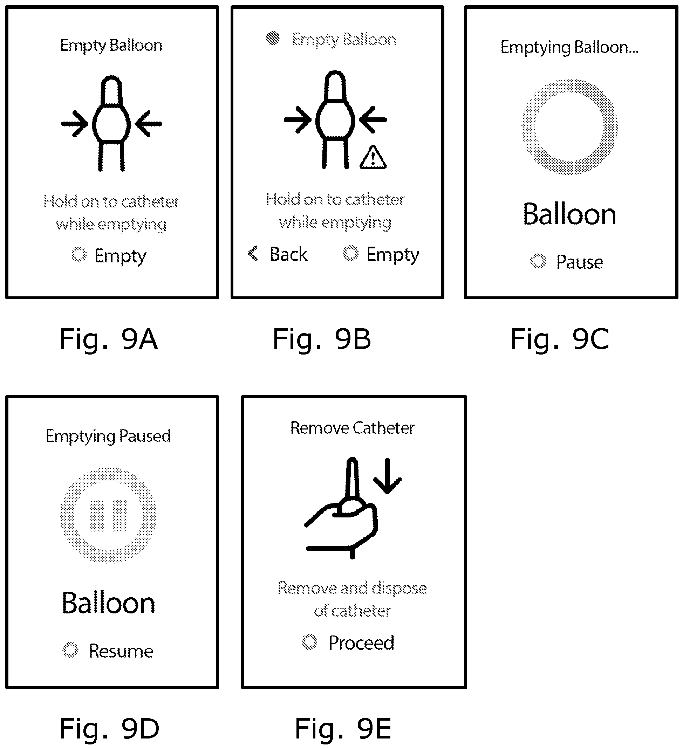

Figs. 9A-9E illustrate displays of a user-operable control interface for controlling operation of the system. In Figs. 9A and 9B, the display provides instructions for the user to hold on to the catheter. As the user activates the "Empty" button, emptying of the balloon (retention element) commences. Fig. 9C illustrates a display shown to the user while emptying is in progress. A pause button is provided allowing the user to pause emptying. A paused emptying state of the system results in the display setting shown in Fig. 9D. Upon completion of emptying, the user is instructed via the display to remove and dispose of the catheter, cf. Fig. 9E.

## Claims

1. A system for anal and/or stomal irrigation comprising:
a reservoir (102) for an irrigating liquid;
a catheter (100) comprising a catheter tip for insertion into the rectum or stoma of a user, the catheter being configured for expelling of the irrigating liquid from the catheter tip, the catheter (100) further comprising an expandable retention element (104) for fixation of the catheter tip within the user's rectum or stoma;
a tubing system providing a first conduit for the irrigating liquid between the reservoir (102) and the catheter (100) and providing a second conduit for the irrigating liquid between the reservoir (102) and the expandable retention element (104);
a valve system in the second conduit for controlling the flow of the irrigating liquid between the reservoir (102) and the expandable retention element (104);
a pump (101) operable to pump the irrigating liquid from the reservoir (102) to the catheter tip, **characterized in that** the pump (101) comprises a reversible pump, which is operable in one direction to pump the irrigating liquid into the expandable retention element (104) for inflation thereof, and which is operable in a reverse direction to withdraw the irrigating liquid from the expandable retention element (104) for collapsing thereof; and
the pump (101) and the valve system being controllable to selectively:
- pump the irrigating liquid into the expandable retention element (104) for expansion thereof;
- pump the irrigating liquid through the catheter (100) for expelling of the irrigating fluid from the catheter tip and into the user's rectum or stoma;
- withdraw the irrigating liquid from the retention element for purging thereof.

2. The system as in claim 1, wherein the pump (101), the valve system and the tubing system are configurable to withdraw the irrigating liquid from the retention element during purging thereof by conveying the irrigating liquid from the retention element directly into the user's rectum or stoma without the irrigating liquid passing into or through the reservoir (102).

3. The system as in any of the preceding claims, wherein the pump (101) is an electrical pump.

4. The system as in any of the preceding claims, wherein the valve system is configured to selectively cause one flow configuration selected from a first, second and third flow configuration at a time, wherein:
- the first flow configuration is arranged to cause a transfer of the irrigating liquid, by means of said pump, from the reservoir (102) into the expandable retention element (104);
- the second flow configuration is arranged to transfer the irrigating liquid, by means of said pump, from the reservoir (102) to the catheter (100);
- the third flow configuration is arranged to transfer the irrigating liquid, by means of said pump, away from the expandable retention element (104).

5. The system as in claim 4, further comprising a first relief valve (110) configured to open if pressure in a bowel of the user exceeds a first threshold limit in the first flow configuration, so as to transfer an amount of the irrigating liquid to the reservoir (102).

6. The system as in claim 4 or 5, further comprising a second relief valve (112) configured to open if pressure in a bowel of the user exceeds a second threshold limit in the second flow configuration, so as to transfer an amount of the irrigating liquid to the reservoir (102).

7. The system as in any of claims 4-6, further comprising:
a first actively controllable valve (106) arranged in the tubing and/or the catheter (100) at a position between the pump (101) and the expandable retention element (104);
a second actively controllable valve (108) arranged in the tubing at a position between the pump (101) and the catheter (100).

8. The system as in any of claims 4-7, further comprising at least one check valve (114,116) for preventing a backflow of the irrigating liquid from the pump (101) in a direction towards the reservoir (102).

9. The system as in any of the preceding claims, further comprising a user-operable control interface for controlling operation of the valve system and/or the pump (101).

10. The system as in any of the preceding claims, wherein the pump (101) is operable to repeatedly expand and collapse the expandable retention element so as to stimulate the peristaltic of the user's bowel.

11. The system as in any of the preceding claims, wherein the expandable retention element (104) is configured to be incrementally expandable or collapsible.

12. The system as in any of the preceding claims, wherein the valve system is operable to redirect the irrigating liquid to the reservoir (102) if the pressure within the expandable retention element (104) exceeds a predetermined threshold level.

13. The system as in any of the preceding claims, wherein the pump (101), the valve system and the tubing system are configurable to withdraw the irrigating liquid from the retention element during purging thereof by conveying the irrigating liquid from the retention element into the reservoir (102).

14. A method of operating a system for anal and/or stomal irrigation, said system comprising:
a reservoir (102) for an irrigating liquid;
a catheter (100) comprising a catheter tip for insertion into the rectum or stoma of a user, the catheter being configured for expelling of the irrigating liquid from the catheter tip, the catheter further comprising an expandable retention element (104) for fixation of the catheter tip within the user's rectum or stoma;
a tubing system providing a first conduit for the irrigating liquid between the reservoir (102) and the catheter (100) and providing a second conduit for the irrigating liquid between the reservoir (102) and the expandable retention element (104);
a valve system in the second conduit for controlling the flow of the irrigating liquid between the reservoir (102) and the expandable retention element (104);
a pump operable to pump the irrigating liquid from the reservoir (102) to the catheter tip **characterized in that** the pump (101) comprises a reversible pump, which is operable in one direction to pump the irrigating liquid into the expandable retention element (104) for inflation thereof, and which is operable in a reverse direction to withdraw the irrigating liquid from the expandable retention element for collapsing thereof;
said method comprising controlling and operating the pump (101) and the valve system to selectively:
- pump the irrigating liquid into the expandable retention element (104) for expansion thereof;
- pump the irrigating liquid through the catheter (100) for expelling of the irrigating fluid from the catheter tip and into the user's rectum or stoma;
- withdraw the irrigating liquid from the retention element for purging thereof.

## Patentansprüche

1. System zur Anal- oder Stoma-Irrigation, umfassend:
ein Reservoir (102) für eine Irrigationsflüssigkeit;
einen Katheter (100), der eine Katheterspitze zum Einführen in das Rektum oder Stoma eines Benutzers umfasst, wobei der Katheter zum Ausstoßen der Irrigationsflüssigkeit aus der Katheterspitze ausgestaltet ist, wobei der Katheter (100) ferner ein expandierbares Halteelement (104) zur Fixierung der Katheterspitze in dem Rektum oder Stoma des Benutzers umfasst,
ein Schlauchsystem, das eine erste Schlauchleitung für die Irrigationsflüssigkeit zwischen dem Reservoir (102) und dem Katheter (100) bereitstellt und eine zweite Schlauchleitung für die Irrigationsflüssigkeit zwischen dem Reservoir (102) und dem expandierbaren Halteelement (104) bereitstellt,
ein Ventilsystem in der zweiten Schlauchleitung zur Steuerung des Stroms der Irrigationsflüssigkeit zwischen dem Reservoir (102) und dem expandierbaren Halteelement (104),
eine Pumpe (101), die dahingehend betätigbar ist, um die Irrigationsflüssigkeit aus dem Reservoir (102) zu der Katheterspitze zu pumpen, **dadurch gekennzeichnet, dass** die Pumpe (101) eine reversierbare Pumpe umfasst, die in einer Richtung betätigbar ist, um die Irrigationsflüssigkeit in das expandierbare Halteelement (104) zu dessen Inflatierung zu pumpen, und die in einer umgekehrten Richtung betätigbar ist, um die Irrigationsflüssigkeit aus dem expandierbaren Halteelement (104) zu saugen, um dies zu kollabieren,
und
wobei die Pumpe (101) und das Ventilsystem steuerbar sind, um gezielt
- die Irrigationsflüssigkeit in das expandierbare Halteelement (104) zu dessen Inflatierung zu pumpen,
- die Irrigationsflüssigkeit durch den Katheter (100) zu pumpen, um die Irrigationsflüssigkeit aus der Katheterspitze und in das Rektum oder das Stoma des Benutzers auszustoßen,
- die Irrigationsflüssigkeit aus dem Halteelement zu saugen, um dies zu leeren.

2. System nach Anspruch 1, wobei die Pumpe (101), das Ventilsystem und das Schlauchsystem dazu ausgestaltbar sind, die Irrigationsflüssigkeit aus dem Halteelement zu saugen, während dieses geleert wird, indem die Irrigationsflüssigkeit aus dem Halteelement direkt in das Rektum oder das Stoma des Benutzers gefördert wird, ohne dass die Irrigationsflüssigkeit in oder durch das Reservoir (102) geht.

3. System nach einem der vorhergehenden Ansprüche, wobei die Pumpe (101) eine elektrische Pumpe ist.

4. System nach einem der vorhergehenden Ansprüche, wobei das Ventilsystem dazu ausgestaltet ist, gezielt zu veranlassen, eine jeweils aus einer ersten, einer zweiten und einer dritten Strömungskonfiguration ausgewählte Strömungskonfiguration zu veranlassen, wobei:
- die erste Strömungskonfiguration dazu angeordnet ist, einen Transfer der Irrigationsflüssigkeit mittels der Pumpe aus dem Reservoir (102) in das expandierbare Halteelement (104) zu veranlassen,
- die zweite Strömungskonfiguration dazu angeordnet ist, die Irrigationsflüssigkeit mittels der Pumpe aus dem Reservoir (102) zu dem Katheter (100) zu transferieren,
- die dritte Strömungskonfiguration dazu angeordnet ist, die Irrigationsflüssigkeit mittels der Pumpe von dem expandierbaren Halteelement (104) weg zu transferieren.

5. System nach Anspruch 4, ferner umfassend ein erstes Entlastungsventil (110), das dazu ausgestaltet ist, sich zu öffnen, wenn Druck in einem Darm des Benutzers einen ersten Schwellenwert in der ersten Strömungskonfiguration überschreitet, um eine Menge der Irrigationsflüssigkeit zu dem Reservoir (102) zu transferieren.

6. System nach Anspruch 4 oder 5, ferner umfassend ein zweites Entlastungsventil (112), das dazu ausgestaltet ist, sich zu öffnen, wenn Druck in einem Darm des Benutzers einen zweiten Schwellenwert in der zweiten Strömungskonfiguration überschreitet, um eine Menge der Irrigationsflüssigkeit zu dem Reservoir (102) zu transferieren.

7. System nach einem der Ansprüche 4 - 6, ferner umfassend:
ein erstes aktiv steuerbares Ventil (106), das in dem Schlauchsystem und/oder dem Katheter (100) an einer Position zwischen der Pumpe (101) und dem expandierbaren Halteelement (104) angeordnet ist,
ein zweites aktiv steuerbares Ventil (108), das in dem Schlauchsystem an einer Position zwischen der Pumpe (101) und dem Katheter (100) angeordnet ist.

8. System nach einem der Ansprüche 4 - 7, ferner umfassend mindestens ein Sperrventil (114, 116), um einen Rückfluss der Irrigationsflüssigkeit von der Pumpe (101) in eine Richtung zu dem Reservoir (102) hin zu verhindern.

9. System nach einem der vorhergehenden Ansprüche, ferner umfassend eine durch den Benutzer betätigbare Bedienungsoberfläche zur Steuerung des Betriebs des Ventilsystems und/oder der Pumpe (101).

10. System nach einem der vorhergehenden Ansprüche, wobei die Pumpe (101) dahingehend betätigbar ist, das expandierbare Halteelement wiederholt zu expandieren und zu kollabieren, um die Peristaltik des Darms des Benutzers zu stimulieren.

11. System nach einem der vorhergehenden Ansprüche, wobei das expandierbare Halteelement (104) schrittweise expandierbar oder kollabierbar ausgestaltet ist.

12. System nach einem der vorhergehenden Ansprüche, wobei das Ventilsystem dahingehend betätigbar ist, die Irrigationsflüssigkeit in das Reservoir (102) umzuleiten, wenn der Druck in dem expandierbaren Halteelement (104) einen vorbestimmten Schwellenwert überschreitet.

13. System nach einem der vorhergehenden Ansprüche, wobei die Pumpe (101), das Ventilsystem und das Schlauchsystem dazu ausgestaltbar sind, die Irrigationsflüssigkeit aus dem Halteelement zu saugen, wenn dieses geleert wird, indem die Irrigationsflüssigkeit aus dem Halteelement in das Reservoir (102) gefördert wird.

14. Verfahren zum Betätigen eines Systems zur Anal- oder Stoma-Irrigation, wobei das System Folgendes umfasst:
ein Reservoir (102) für eine Irrigationsflüssigkeit;
einen Katheter (100), der eine Katheterspitze zum Einführen in das Rektum oder Stoma eines Benutzers umfasst, wobei der Katheter zum Ausstoßen der Irrigationsflüssigkeit aus der Katheterspitze ausgestaltet ist, wobei der Katheter ferner ein expandierbares Halteelement (104) zur Fixierung der Katheterspitze in dem Rektum oder Stoma des Benutzers umfasst,
ein Schlauchsystem, das eine erste Schlauchleitung für die Irrigationsflüssigkeit zwischen dem Reservoir (102) und dem Katheter (100) bereitstellt und eine zweite Schlauchleitung für die Irrigationsflüssigkeit zwischen dem Reservoir (102) und dem expandierbaren Halteelement (104) bereitstellt,
ein Ventilsystem in der zweiten Schlauchleitung zur Steuerung des Stroms der Irrigationsflüssigkeit zwischen dem Reservoir (102) und dem expandierbaren Halteelement (104),
eine Pumpe, die dahingehend betätigbar ist, um die Irrigationsflüssigkeit aus dem Reservoir (102) zu der Katheterspitze zu pumpen, **dadurch gekennzeichnet, dass** die Pumpe (101) eine reversierbare Pumpe umfasst, die in einer Richtung betätigbar ist, um die Irrigationsflüssigkeit in das expandierbare Halteelement (104) zu dessen Inflatierung zu pumpen, und die in einer umgekehrten Richtung betätigbar ist, um die Irrigationsflüssigkeit aus dem expandierbaren Halteelement zu saugen, um dies zu kollabieren, wobei das Verfahren das Steuern und Betätigen der Pumpe (101) und des Ventilsystems umfasst, um gezielt
- die Irrigationsflüssigkeit in das expandierbare Halteelement (104) zu dessen Inflatierung zu pumpen,
- die Irrigationsflüssigkeit durch den Katheter (100) zu pumpen, um die Irrigationsflüssigkeit aus der Katheterspitze und in das Rektum oder das Stoma des Benutzers auszustoßen,
- die Irrigationsflüssigkeit aus dem Halteelement zu saugen, um dies zu leeren.

## Revendications

1. Système pour irrigation anale et/ou de stomie, comprenant :
un réservoir (102) pour un liquide d'irrigation ;
un cathéter (100) comprenant une pointe de cathéter prévue pour être insérée dans le rectum ou la stomie d'un utilisateur, le cathéter étant configuré pour expulser le liquide d'irrigation depuis la pointe de cathéter, le cathéter (100) comprenant en outre un élément de retenue expansible (104) prévu pour fixer la pointe de cathéter à l'intérieur du rectum ou de la stomie de l'utilisateur ;
un système de tubes fournissant un premier conduit pour le liquide d'irrigation entre le réservoir (102) et le cathéter (100) et fournissant un deuxième conduit pour le liquide d'irrigation entre le réservoir (102) et l'élément de retenue expansible (104) ;
un système de soupape dans le deuxième conduit pour réguler l'écoulement du liquide d'irrigation entre le réservoir (102) et l'élément de retenue expansible (104) ;
une pompe (101) capable de pomper le liquide d'irrigation depuis le réservoir (102) vers la pointe de cathéter, **caractérisé en ce que** la pompe (101) comprend une pompe réversible, qui peut fonctionner dans une direction pour pomper le liquide d'irrigation dans l'élément de retenue expansible (104) en vue de gonfler ce dernier, et qui peut fonctionner dans une direction inverse pour extraire le liquide d'irrigation de l'élément de retenue expansible (104) en vue de dégonfler ce dernier ; et
la pompe (101) et le système de soupape pouvant être commandés de manière sélective pour :
- pomper le liquide d'irrigation dans l'élément de retenue expansible (104) en vue de gonfler ce dernier ;
- pomper le liquide d'irrigation à travers le cathéter (100) pour expulser le fluide d'irrigation depuis la pointe de cathéter et dans le rectum ou la stomie de l'utilisateur ;
- extraire le liquide d'irrigation de l'élément de retenue en vue de purger ce dernier.

2. Système selon la revendication 1, dans lequel la pompe (101), le système de soupape et le système de tubes peuvent être configurés pour extraire le liquide d'irrigation de l'élément de retenue au cours de la purge de ce dernier en transportant le liquide d'irrigation depuis l'élément de retenue directement dans le rectum ou la stomie de l'utilisateur sans que le liquide d'irrigation ne passe dans ou à travers le réservoir (102).

3. Système selon l'une quelconque des revendications précédentes, dans lequel la pompe (101) est une pompe électrique.

4. Système selon l'une quelconque des revendications précédentes, dans lequel le système de soupape est configuré de manière sélective pour engendrer à un moment donné une configuration d'écoulement choisie parmi une première, une deuxième et une troisième configuration d'écoulement, dans lesquelles :
- la première configuration d'écoulement est prévue pour causer un transfert du liquide d'irrigation au moyen de ladite pompe depuis le réservoir (102) dans l'élément de retenue expansible (104) ;
- la deuxième configuration d'écoulement est prévue pour transférer le liquide d'irrigation au moyen de ladite pompe depuis le réservoir (102) vers le cathéter (100) ;
- la troisième configuration d'écoulement est prévue pour transférer le liquide d'irrigation au moyen de ladite pompe à l'écart de l'élément de retenue expansible (104).

5. Système selon la revendication 4, comprenant en outre une première soupape de détente (110) configurée pour s'ouvrir si la pression dans l'intestin de l'utilisateur dépasse une première limite seuil dans la première configuration d'écoulement de manière à transférer une quantité du liquide d'irrigation vers le réservoir (102).

6. Système selon la revendication 4 ou 5, comprenant en outre une deuxième soupape de détente (112) configurée pour s'ouvrir si la pression dans l'intestin de l'utilisateur dépasse une deuxième limite seuil dans la deuxième configuration d'écoulement de manière à transférer une quantité du liquide d'irrigation vers le réservoir (102).

7. Système selon l'une quelconque des revendications 4 à 6, comprenant en outre :
une première soupape pouvant être commandée activement (106) disposée dans le tube et/ou dans le cathéter (100) à une position entre la pompe (101) et l'élément de retenue expansible (104) ;
une deuxième soupape pouvant être commandée activement (108) disposée dans le tube à une position entre la pompe (101) et le cathéter (100) .

8. Système selon l'une quelconque des revendications 4 à 7, comprenant en outre au moins un clapet antiretour (114, 116) pour empêcher un reflux du liquide d'irrigation depuis la pompe (101) dans une direction vers le réservoir (102).

9. Système selon l'une quelconque des revendications précédentes, comprenant en outre une interface de commande pouvant être commandée par un utilisateur pour commander le fonctionnement du système de soupape et/ou de la pompe (101).

10. Système selon l'une quelconque des revendications précédentes, dans lequel la pompe (101) peut être commandée pour gonfler et dégonfler de manière répétée l'élément de retenue expansible de manière à stimuler le péristaltisme de l'intestin de l'utilisateur.

11. Système selon l'une quelconque des revendications précédentes, dans lequel l'élément de retenue gonflable (104) est configuré pour pouvoir être gonflé ou dégonflé de manière incrémentale.

12. Système selon l'une quelconque des revendications précédentes, dans lequel le système de soupape peut fonctionner pour rediriger le liquide d'irrigation vers le réservoir (102) si la pression à l'intérieur de l'élément de retenue gonflable (104) dépasse un niveau seuil prédéterminé.

13. Système selon l'une quelconque des revendications précédentes, dans lequel la pompe (101), le système de soupape et le système de tubes peuvent être configurés pour extraire le liquide d'irrigation de l'élément de retenue au cours de la purge de ce dernier en transportant le liquide d'irrigation depuis l'élément de retenue dans le réservoir (102).

14. Procédé pour faire fonctionner un système pour irrigation anale et/ou de stomie, ledit système comprenant :
un réservoir (102) pour un liquide d'irrigation ;
un cathéter (100) comprenant une pointe de cathéter prévue pour être insérée dans le rectum ou la stomie d'un utilisateur, le cathéter étant configuré pour expulser le liquide d'irrigation depuis la pointe de cathéter, le cathéter comprenant en outre un élément de retenue expansible (104) prévu pour fixer la pointe de cathéter à l'intérieur du rectum ou de la stomie de l'utilisateur ;
un système de tubes fournissant un premier conduit pour le liquide d'irrigation entre le réservoir (102) et le cathéter (100) et fournissant un deuxième conduit pour le liquide d'irrigation entre le réservoir (102) et l'élément de retenue expansible (104) ;
un système de soupape dans le deuxième conduit pour réguler l'écoulement du liquide d'irrigation entre le réservoir (102) et l'élément de retenue expansible (104) ;
une pompe capable de pomper le liquide d'irrigation depuis le réservoir (102) vers la pointe de cathéter, **caractérisé en ce que** la pompe (101) comprend une pompe réversible, qui peut fonctionner dans une direction pour pomper le liquide d'irrigation dans l'élément de retenue expansible (104) en vue de gonfler ce dernier, et qui peut fonctionner dans une direction inverse pour extraire le liquide d'irrigation de l'élément de retenue expansible en vue de dégonfler ce dernier ; ledit procédé comprenant la commande et l'opération de la pompe (101) et du système de soupape pour, de manière sélective :
- pomper le liquide d'irrigation dans l'élément de retenue gonflable (104) en vue de gonfler ce dernier ;
- pomper le liquide d'irrigation à travers le cathéter (100) pour expulser le fluide d'irrigation depuis la pointe de cathéter et dans le rectum ou la stomie de l'utilisateur ;
- extraire le liquide d'irrigation de l'élément de retenue en vue de purger ce dernier.
